Europäisches Patentamt

European Patent Office

Office européen des brevets

(19)

(11) Publication number: **0 002 314**
A1

(12) **EUROPEAN PATENT APPLICATION**

(21) Application number: **78300547.3**

(22) Date of filing: **26.10.78**

(51) Int. Cl.²: **C 07 C 39/04**
**C 07 C 37/38**

(30) Priority: **07.11.77 GB 46213/77**

(43) Date of publication of application:
**13.06.79 Bulletin 79/12**

(84) Designated contracting states:
**BE DE FR GB NL**

(71) Applicant: **IMPERIAL CHEMICAL INDUSTRIES LIMITED**
**Imperial Chemical House Millbank**
**London SW1P 3JF(GB)**

(72) Inventor: **Featherstone, William**
**18 Gypsy Lane**
**Nunthorpe Middlesbrough Cleveland(GB)**

(72) Inventor: **Hobson, Geoffrey Keith**
**28 Enfield Chase**
**Hunters Hill Guisborough Cleveland(GB)**

(74) Representative: **Locke, Timothy John et al,**
**Imperial Chemical Industries Limited Legal Department:**
**Patents Thames House North Millbank**
**London SW1P 4QG(GB)**

(54) **Separation of phenol.**

(57) Phenol is separated from mixtures comprising phenol and cumene and/or α-methylstyrene by distillation in the presence of water in a column to which cumene and/or α-methylstyrene and water are fed as reflux in quantities sufficient to produce two liquid phases in the upper part of the column.

EP 0 002 314 A1

Separation of Phenol

THIS INVENTION relates to the separation of phenol.

Phenol is commonly manufactured from cumene by oxidising the cumene to cumene hydroperoxide and decomposing the cumene hydroperoxide to phenol and acetone.    In processes for manufacturing phenol of this type streams may be produced comprising phenol together with cumene and/or α-methylstyrene.   Cumene may be recovered and re-used in the process and α-methylstyrene may also be recovered and re-used in the process after reduction to cumene.

This invention comprises a process of separating phenol from a mixture comprising phenol and cumene and/or α-methylstyrene which comprises distilling the mixture in the presence of water in a distillation column, feeding to the top of the column a reflux comprising cumene and/ or α-methylstyrene and also water in quantities sufficient to produce two liquid phases in the upper part of the distillation column and recovering phenol from the base of the column and cumene and/or α-methylstyrene and water from the top of the column.

The cumene and/or α-methylstyrene and water recovered from the top of the column separate on condensation into  two liquid phases.   It is  preferred that at least part of the water fed to the top of the column should be from the water rich phase produced by this condensation; the cumene and/or α-methylstyrene may be supplied from the cumene and/or α-methylstyrene rich phase from the condensation.

Water may be present in the mixture when it is fed to the column.  If desired water may also be fed preferably in the form of steam into a part of the column below that at which the mixture is fed.

The mixture fed to the column may comprise 20-95% and preferably 50-95% by weight of phenol, 10-40% and preferably 15-30% by weight of cumene and/or α-methylstyrene and 1-60% and more preferably 5-20% by weight of water.

The process is particularly  suitable for separating mixtures containing high molar ratio of cumene to α-methyl-styrene, for example greater than 3 to 1, but may be used at advantageously at any ratio of cumene to α-methylstyrene.

The total water fed to the column (as steam, with the reflux and as a component of the mixture fed to the column) will bear the same ratio to the total of cumene and/or α-methylstyrene fed to the column (as reflux and in the mixture) as does the water to the cumene and/or α-methylstyrene taken as overhead vapour from the column . This ratio will correspond to the composition of the heteroazeotrope of water with cumene and/or α-methylstyrene and is approximately 44% by weight of water and 56% by weight of cumene and/or α-methylstyrene if the distillation is conducted at substantially atmospheric pressure.  Suitably the ratio by weight of cumene and/or α-methylstyrene to water fed to the top of the column is in the range 1:2 to 9:1 and is more preferably 2:3 to 9:1.  These ratios may be adjusted in accordance with the desired reflux ratio and the amounts of water and cumene and/or α-methylstyrene intro-duced with the mixture and as steam.

The composition of the heteroazeotrope of water with cumene and/or α-methylstyrene varies little with pressure.

It is preferred that the distillation should be carried out at a pressure in the range 0.1 - 50 bars absolute and more preferably 0.2 - 10 bars absolute.

The column suitably comprise: 20 - 100 theoretical plates and the molar reflux ratio may be for example in the range 0.2 to 10     and is preferably in the range 1 to 5.

EXAMPLE 1

A mixture comprising 78.2% by weight phenol, 14.1% by weight cumene, 2.0% by weight α-methylstyrene and 7.9% by weight water was fed at a temperature of 60-70°C to a glass Oldershaw column with 100 actual plates (approximately 50 theorectical plates), at the actual plate 40 from the bottom of the column.   The pressure at the top of the column was 1 atmosphere and distillation was carried out at a reflux ratio of 3.0 on a molar basis.

The overheads from the column were condensed to a two-phase mixture.   If the organic phase from the condensate only was used as reflux the phenol content of the organic layer of the top product was approximately 5.4% by weight and that of the aqueous layer 2% by weight. The phenol recovered as a bottoms product from the column contained 6 parts per million by weight of cumene and 5 parts per million by weight of a α-methylstyrene.

When the reflux was 6.5 moles of the aqueous phase to one mole of the organic phase the phenol content of the organic layer of the top product was 0.7% by weight and that of the aqueous layer 0.5% by weight. The phenol recovered as a bottoms product contained 4 parts per million by weight cumene and 3 parts per million by weight of α-methylstyrene.

EXAMPLE 2

A mixture comprising 67% by weight phenol, 22.7% by weight cumene, 3.8% by weight α-methylstyrene and 6.5% by weight water was fed at a temperature of 60-70°C to a glass Oldershaw column with 100 actual plates (approximately 50 theorectical plates), at the actual plate 40 from the bottom of the column.   The pressure at the top of the column was 1 atmosphere. Distillation was carried out at an overall reflux ratio of 4.8 on a molar basis and the reflux contained a ratio of 7.6 moles of the aqueous phase of the condensate to one mole of the organic phase.   I

phenol content of the organic layer of the top product was 0.03% by weight and that of the aqueous layer 0.06% by weight.    The phenol recovered as a bottoms product contained 3 parts per million by weight cumene and 43 parts per million by weight of α-methylstyrene.

EXAMPLE 3

A mixture comprising 67% by weight phenol, 22.7% by weight cumene, 3.8% by weight α-methylstyrene and 6.5% by weight water was fed at a temperature of 60 - 70°C to a 2 inch diameter glass column packed with Knitmesh packing. The total height of the packing was 119.5 inches with 69.5 inches  below the point of entry of the mixture.  The pressure at the top of the column was 1 atmosphere. Distillation was carried out an an overall reflux ratio of 2.2 on a molar basis and the reflux contained a ratio of 9.6 moles of the aqueous phase of the condensate to one mole of the organic phase.  The phenol content of the organic layer of the top product was 0.25% by weight and that of the aqueous layer 0.2% by weight.  The phenol recovered as a bottoms product contained 18 parts per million by weight cumene and 50 parts per million by weight α-methylstyrene.

Liquid aqueous layer which separates from the condensate is substantially free from α-methyl styrene and cumene and the organic layer is substantially free from water in each of the above examples.

Claims

1. A process of separating phenol from mixtures comprising phenol and cumene and/or α-methylstyrene which comprises distilling the mixture in the presence of water in a distilla - tion column, feeding to the top of the column a reflux comprising cumene and/or α-methylstyrene and also water in quantities sufficient to produce two liquid phases in the upper part of the distillation column and recovering phenol from the base of the column and cumene and/or α-methylstyrene and water from the top of the column.

2. A process as claimed in Claim 1 in which the cumene and/or α-methylstyrene and water recovered from the top of the column is condensed, separated into two liquid phases and the cumene and/or α-methylstyrene fed to the top of the column is supplied from the cumene and/or α-methylstyrene rich phase and at least part of the water fed to the top of the column is supplied from the water rich phase.

3. A process as claimed in Claim 1 or 2 in which water is present in the mixture when it is fed to the column.

4. A process as claimed in Claim 1, 2 or 3 in which the mixture fed to the column comprises 50 to 95% by weight of phenol.

5. A process as claimed in any of the preceding claims in which the mixture fed to the column comprises 15 to 30% by weight of cumene and/or α-methylstyrene.

6. A process as claimed in any of the preceding claims in which the mixture fed to the column comprises 5 to 20% by weight of water.

7. A process as claimed in any of the preceding claims in which the mixture fed to the column comprises cumene and α-methylstyrene in a molar ratio greater than 3 : 1.

8.   A process as claimed in any of the preceding claims in which the ratio by weight of cumene and/or α-methyl-styrene to water fed to the top of the column is in the range 2 : 3 to 9 : 1 and in which the distillation is carried out at a pressure in the range 0.2 to 10 bars absolute.

9.   A process as claimed in any of the preceding claims in which the column comprises 20 to 100 theoretical plates.

10.  A process as claimed in any of the preceding claims in which the distillation is carried out at a molar reflux ratio in the range 1 to 5.

0002314

EUROPEAN SEARCH REPORT

European Patent Office

Application number

EP 78 30 0547

## DOCUMENTS CONSIDERED TO BE RELEVANT

| Category | Citation of document with indication, where appropriate, of relevant passages | Relevant to claim |
|---|---|---|
| X | <u>FR - A - 1 114 955</u> (RUETGERSWERKE)<br>* Pages 2-4; figure * | 1-3,8, 10 |
| | --- | |
| X | <u>GB - A - 770 650</u> (RUETGERSWERKE)<br>* Complete patent * | 1-3,8 |
| | --- | |
| X | <u>GB - A - 768 941</u> (RUETGERSWERKE)<br>* Example 6; figure * | 1,2. |
| | --- | |
| | <u>BE - A - 524 951</u> (THE DISTILLERS COMPANY)<br>* Pages 5-7; figure 2 * | 1-4,6, 8-10 |
| | ---- | |

CLASSIFICATION OF THE APPLICATION (Int. Cl.²)

C 07 C 39/04
37/38

TECHNICAL FIELDS SEARCHED (Int.Cl.²)

C 07 C 37/38

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 19-01-1979 | VAN GEYT |

EPO Form 1503.1   06.78